# EUROPÄISCHE PATENTSCHRIFT

(11) **EP 0 559 695 B1**
(45) Veröffentlichungstag und Bekanntmachung des Hinweises auf die Patenterteilung: **22.01.1997**
(21) Anmeldenummer: 91920436.2
(22) Anmeldetag: 28.11.1991
(51) Int. Cl.: C07D 401/12, C07D 213/57, C07D 213/55, C07D 213/30, A61K 31/44

(54) **NEUE PYRIDINDERIVATE, VERFAHREN ZU IHRER HERSTELLUNG UND VERWENDUNG ALS ARZNEIMITTEL**
NEW PYRIDINE DERIVATIVES, METHODS OF PREPARING THEM AND THEIR USE AS DRUGS
NOUVEAUX DERIVES DE PYRIDINE, LEUR PROCEDE DE PRODUCTION ET LEUR UTILISATION COMME MEDICAMENTS

(30) Priorität: 01.12.1990 DE 4038335
(43) Veröffentlichungstag der Anmeldung: 15.09.1993
(73) Patentinhaber: BOEHRINGER MANNHEIM GMBH, 68298 Mannheim (DE)
(72) Erfinder: FRIEBE, Walter-Gunar, D-6800 Mannheim 1 (DE); KAMPE, Wolfgang, D-6805 Heddesheim (DE); LINSSEN, Marcel, D-6712 Bobenheim-Roxheim (DE); WILHELMS, Otto-Henning, D-6941 Weinheim-Rittenweier (DE)
(74) Vertreter: Weber, Manfred, Dr.
(86) Internationale Anmeldenummer: EP9102249
(87) Internationale Veröffentlichungsnummer: WO9209598

(56) Entgegenhaltungen:
- EP-A- 0 318 083
- EP-A- 0 384 450
- WO-A-88/04305
- FR-A- 2 445 319
- JOURNAL OF MEDICINAL CHEMISTRY. Bd. 27, Nr. 2, Februar 1984, WASHINGTON US Seiten 125 - 128; Y. HONMA ET AL.: 'ANTIALLERGIC AGENTS. 3. N-(1H-TETRAZOL-5- YL)-2-PYRIDINECARBOXAMIDES.' Siehe Verbindungen 14, 39, 40.
- CHEMICAL ABSTRACTS, vol. 114, no. 7, 18. Februar 1991, Columbus, Ohio, US; abstract no. 61941B, T. HAMAZAKI ET AL.: 'Preparation of pyridines as leukotriene inhibitors.' Seite 674 ;
- CHEMICAL ABSTRACTS, vol. 103, no. 25, 23. Dezember 1985, Columbus, Ohio, US; abstract no. 215180, 'Pyridine and pyrimidine derivatives as antiallergics.' Seite 883 ;

## Beschreibung

Die vorliegende Erfindung betrifft neue Pyridinderivate der allgemeinen Formel I 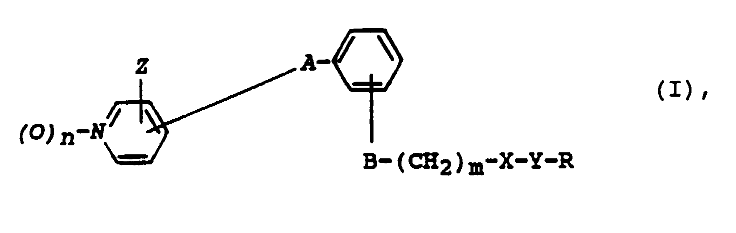 in welcher
- A: eine Gruppe -CH=CH-,
- B: ein Sauerstoffatom oder eine Valenzbindung,
- m: eine ganze Zahl von 0 bis 5,
- n: die Zahl 0 oder 1,
- X: eine Valenzbindung, ein Sauerstoffatom oder ein Schwefelatom,
- Y: eine Valenzbindung,
- Z: Wasserstoff, Halogen, C₁- bis C₆-Alkyl oder Cyano und
- R: eine Gruppe CN, COOH, COOC₁- bis C₆-Alkyl, CONH-Tetrazolyl, CON(OH)(C₁-C₆-Alkyl) oder 5-(1H)-Tetrazolyl,
mit Ausnahme der Verbindung 4-[2-(4)-Pyridinovinyl]-carboxymethoxybenzol, mit der Maßgabe, daß wenn R eine Gruppe CN, CO-NH-Tetrazolyl und B, m, n, X, Y und Z die angegebene Bedeutung haben, A auch einen Alkylenrest mit 1 bis 3 Kohlenstoffatomen darstellen kann,
sowie deren physiologisch verträgliche Salze und Verfahren zu ihrer Herstellung sowie diese Verbindungen enthaltende Arzneimittel.

Ferner sind Gegenstand der vorliegenden Erfindung Verbindungen der Formel I, in der
- A: einen Alkylenrest mit 1 bis 3 Kohlenstoffatomen oder eine Gruppe -CH=CH-,
- B: ein Sauerstoffatom oder eine Valenzbindung,
- m: eine ganze Zahl von 0 bis 5,
- n: die Zahl 0 oder 1,
- X: eine Valenzbindung, ein Sauerstoffatom oder ein Schwefelatom,
- Y: einen gewünschtenfalls ein- oder mehrfach durch C₁-C₆-Alkyl, C₁-C₆-Alkoxy, Halogen, Hydroxy oder Aminocarbonyl substituierten Phenylenrest,
- Z: Wasserstoff, Halogen, C₁- bis C₆-Alkyl oder Cyano und
- R: eine Gruppe CN, COOH, COOC₁- bis C₆-Alkyl, CONH-Tetrazolyl, CON(OH)(C₁-C₆-Alkyl) oder 5-(1H)-Tetrazolyl bedeuten,
sowie deren physiologisch verträgliche Salze.

Verbindungen der Formel I, in der A = eine Alkylenkette, B = ein Sauerstoffatom oder ein Valenz, m = eine ganze Zahl von 0 bis 5, n = 0, X = Valenz und Y = Valenz, Z = Wasserstoff oder Alkyl, R = CN, COOH und COOAlk sind teilweise in DE-A- 2 951 786 als Thromboxan-Synthetase-Hemmer beschrieben. In der DE-A- 3 641 024 ist die Verbindung 4-[2-(4)-Pyridinovinyl]-carboxymethoxybenzol namentlich aufgeführt, die als Flüssigkristall-Baustein Verwendung findet.

In J. Med. Chem 1989, 27, S. 125 - 128 und Pyridylethenylphenyl-Derivaten mit antiallergischer Wirkung beschrieben, jedoch ist der Phenylring unsubstituiert.

Ferner sind in Chem. Abstr., Bd. 114, No. 7 1991, 61 941 (b) Pyridylethenyl-phenyl-Derivate beschrieben, wobei der Pyridyl-Ring substituiert sein muß.

Die neuen Verbindungen der allgemeinen Formel I weisen wertvolle pharmakologische Eigenschaften auf, insbesondere können sie die antigenbedingte Kontraktion von Lungengewebestreifen hemmen. Sie eignen sich daher zur Behandlung allergischer Krankheiten sowie von entzündungsbedingten bronchospastischen und bronchokonstriktorischen Reaktionen.

Die Alkylreste in den genannten Gruppen können geradkettig oder verzweigt sein. Bevorzugte Alkylreste sind der Methyl-, Ethyl-, n-Propyl-, Isopropyl-, n-Butyl-, Isobutyl-, tert.-Butyl-, n-Pentyl- und 3-Pentylrest.

Halogenatome sind insbesondere Fluor, Chlor und Brom.

Die Bindung des Restes A kann in 2-, 3- oder 4-Stellung des Pyridinrings, des Restes B in 2-, 3- oder 4-Stellung des Phenylrings erfolgen.

Besonders bevorzugt sind Verbindungen der Formel I, in denen A, B, m, n, X, Y und Z die angegebene Bedeutung haben, und R eine Gruppe CO-NH-Tetrazolyl, -CON(OH)(C₁-C₆-Alkyl) oder 5-(1H)-Tetrazolyl bedeuten.

Ferner sind Verbindungen der Formel I bevorzugt, in denen A, B, m, n, Y, Z und R die angegebene Bedeutung haben, und Y einen gewünschtenfalls substituierten Phenylenrest darstellt.

Außer den in den Beispielen genannten Verbindungen sind Gegenstand der vorliegenden Erfindung insbesondere alle Substanzen, die jede mögliche Kombination der in den Beispielen genannten Substituenten aufweisen.

Das erfindungsgemäße Verfahren zur Herstellung der Verbindungen der Formel I ist dadurch gekennzeichnet, daß man in an sich bekannter Weise entweder
a) eine Verbindung der allgemeinen Formel II 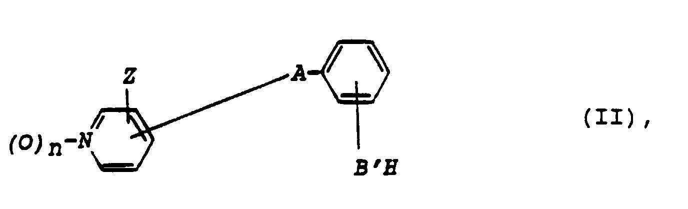 in welcher
A, Z und n die angegebene Bedeutung haben und B' ein Sauerstoffatom bedeutet,
mit einer Verbindung der allgemeinen Formel III

G-(CH₂)ₘ-X-Y-R (III),

in welcher
X, Y, R und m die angegebene Bedeutung haben und G einen reaktiven Rest darstellt, umsetzt, oder
b) eine Verbindung der allgemeinen Formel IV 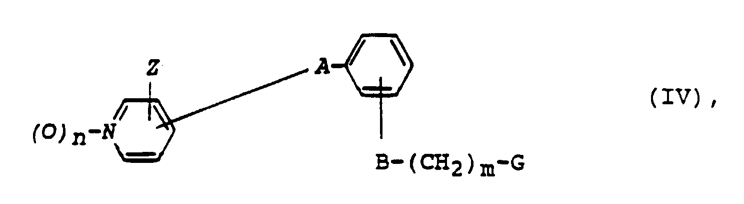 in welcher A, B, G, Z, m und n die angegebene Bedeutung haben,
mit einer Verbindung der allgemeinen Formel V

H-X-Y-R (V),

in welcher
R, X und Y die angegebene Bedeutung haben,
umsetzt, oder
c) für den Fall, daß A die Gruppe -CH=CH- bedeutet, eine Verbindung der allgemeinen Formel VI 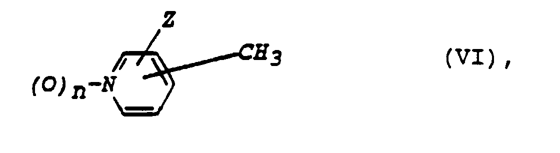 in welcher
Z und n die angegebene Bedeutung haben,
mit einer Verbindung der allgemeinen Formel VII 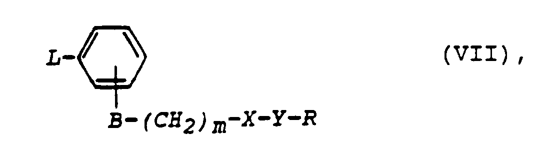 in welcher
B, X, Y, R und m die angegebene Bedeutung haben und L eine Formylgruppe oder ein reaktives Derivat hiervon darstellt,
umsetzt
und anschließend gewünschtenfalls einen Substituenten R in einen anderen, durch die Definition gegebenen Substituenten R oder eine Gruppe A in eine andere durch die Definition gegebene Gruppe A überführt oder den Pyridinstickstoff oxidiert und die erhaltene Verbindung der Formel I gewünschtenfalls durch Umsetzung mit physiologisch verträglichen Säuren oder Basen in ein Salz umwandelt.

Als reaktive Reste G kommen Chlor, Brom, Mesyloxy oder Tosyloxy in Frage. Eine Umwandlung eines Restes R in einen anderen, durch den Anspruch definierten Rest R erfolgt beispielsweise durch Umsetzung einer Verbindung der Formel I, in der R für eine Cyanogruppe steht, mit Stickstoffwasserstoffsäure oder einem Metallazid und einer protonenliefernden Substanz wie beispielsweise Ammoniumchlorid zum Tetrazolylrest oder mit wäßrigen Säuren oder Laugen zum Carboxylrest. Eine für R stehende Carboxylgruppe kann, gewünschtenfalls über ein reaktives Derivat wie ein Säurehalogenid, Säureanhydrid oder Imidazolid, verestert oder amidiert werden; aus einer für R stehenden Alkoxycarbonylgruppe läßt sich durch saure oder basische Hydrolyse die Carbonsäure, durch Aminolyse ein Carboxamid erhalten.

Die Umsetzung von Verbindungen der Formeln II und III bzw. IV und V erfolgt zweckmäßig in neutralem oder basischem Medium, beispielsweise in Wasser oder einem niederen Alkohol wie Methanol, Ethanol oder Isopropanol in Gegenwart eines Alkalialkoholats oder Alkalihydroxids oder in aprotischen Lösungsmitteln wie Aceton, Butanon oder Dimethylformamid in Gegenwart eines säurebindenden Mittels wie Natriumcarbonat, Kaliumcarbonat oder Calciumcarbonat bei Temperaturen zwischen 0°C und dem Siedepunkt des Reaktionsgemisches.

Die Reaktion von Verbindungen der Formeln VI und VII führt man ohne Lösungsmittel bei erhöhter Temperatur, gewünschtenfalls unter Druck, oder in Gegenwart eines wasserbindenden Mittels wie konzentrierte Salzsäure oder Essigsäureanhydrid unter Erwärmung durch.

Die Ausgangsverbindungen II bis VII sind literaturbekannte Substanzen oder können in Analogie zu literaturbekannten Verfahren hergestellt werden.

Als pharmakologisch verträgliche Salze kommen insbesondere Alkali-, Erdalkali- und Ammoniumsalze sowie gegebenenfalls Salze mit nichttoxischen anorganischen oder organischen Säuren, wie z.B. Salzsäure, Schwefelsäure, Phosphorsäure, Bromwasserstoffsäure, Essigsäure, Milchsäure, Zitronensäure, Äpfelsäure, Benzoesäure, Salicylsäure, Malonsäure, Maleinsäure, Bernsteinsäure oder Diaminocapronsäure in Frage.

Die Salze erhält man in üblicher Weise z.B. durch Neutralisation der Verbindungen der Formel I mit den entsprechenden Laugen oder Säuren.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und / oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen I auch in Form von Pudern und Salben verwendet werden. Sie werden dazu z.B. mit pulverförmigen, physiologisch verträglichen Verdünnungsmitteln bzw. üblichen Salbengrundlagen vermischt.

Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Außer den in den Beispielen genannten Substanzen sind im Sinne der vorliegenden Erfindung die folgenden Verbindungen bevorzugt:
4-<4-[2-(2-Pyridinyl)ethenyl]phenylmethoxy>benzonitril
4-<2-[2-(2-Pyridinyl)ethenyl]phenylmethoxy>benzonitril
4-<4-[2-(4-Pyridinyl)ethyl]phenylmethoxy>benzonitril
4-[2-(4-Phenoxymethyl-phenyl)ethyl]pyridin
4-<2-[4-(4-Chlor-phenoxymethyl)phenyl]ethenyl>pyridin
4-<2-[4-(4-Methoxy-phenoxymethyl)phenyl]ethenyl>pyridin
4-<2-[4-(4-Methyl-phenoxymethyl)phenyl]ethenyl>pyridin
4-<2-[4-(3,4-Dichlor-phenoxymethyl)phenyl]ethenyl>pyridin
4-<2-[4-(4-Hydroxy-phenoxymethyl)phenyl]ethenyl>pyridin
5-{4-<4-[2-(4-Pyridinyl)ethenyl]phenylmethoxy>phenyl}-1H-tetrazol
4-<4-[2-(4-Pyridinyl)ethenyl]phenylmethoxy>benzamid
4-<4-[2-(4-Pyridinyl)ethenyl]phenylmethoxy>benzoesäure-N-(1H-tetrazol-5-yl)amid
4-<4-[2-(4-N-Oxido-pyridinyl)ethyl]phenoxy>buttersäure-N-(1H-tetrazol-5-yl)amid

### Beispiel 1

### 5-<4-[2-(4-Pyridinyl)ethenyl]phenoxy>valeronitril

Zu der Lösung von 1.16 g (50 mmol) Natrium in 250 ml 2-Propanol gibt man 10.0 g (50 mmol) 4-[2-(4-Pyridinyl)ethenyl]phenol, erwärmt 10 min zum Rückfluß, läßt abkühlen, fügt 8.1 g (50 mmol) 5-Bromvaleronitril zu und erhitzt 16 h zum Rückfluß. Man engt ein, versetzt mit Wasser, extrahiert mit Dichlormethan, trocknet den Extrakt, engt ein und verreibt den Rückstand mit Ether. Man erhält 11.9 g Titelverbindung (86 % d. Th.) vom Schmp. 85-87°C.

### Beispiel 2

In analoger Weise wie in Beispiel 1 beschrieben erhält man:

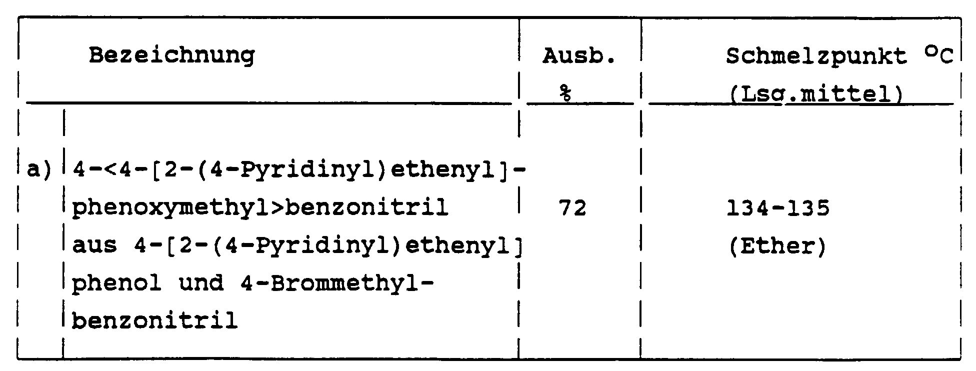

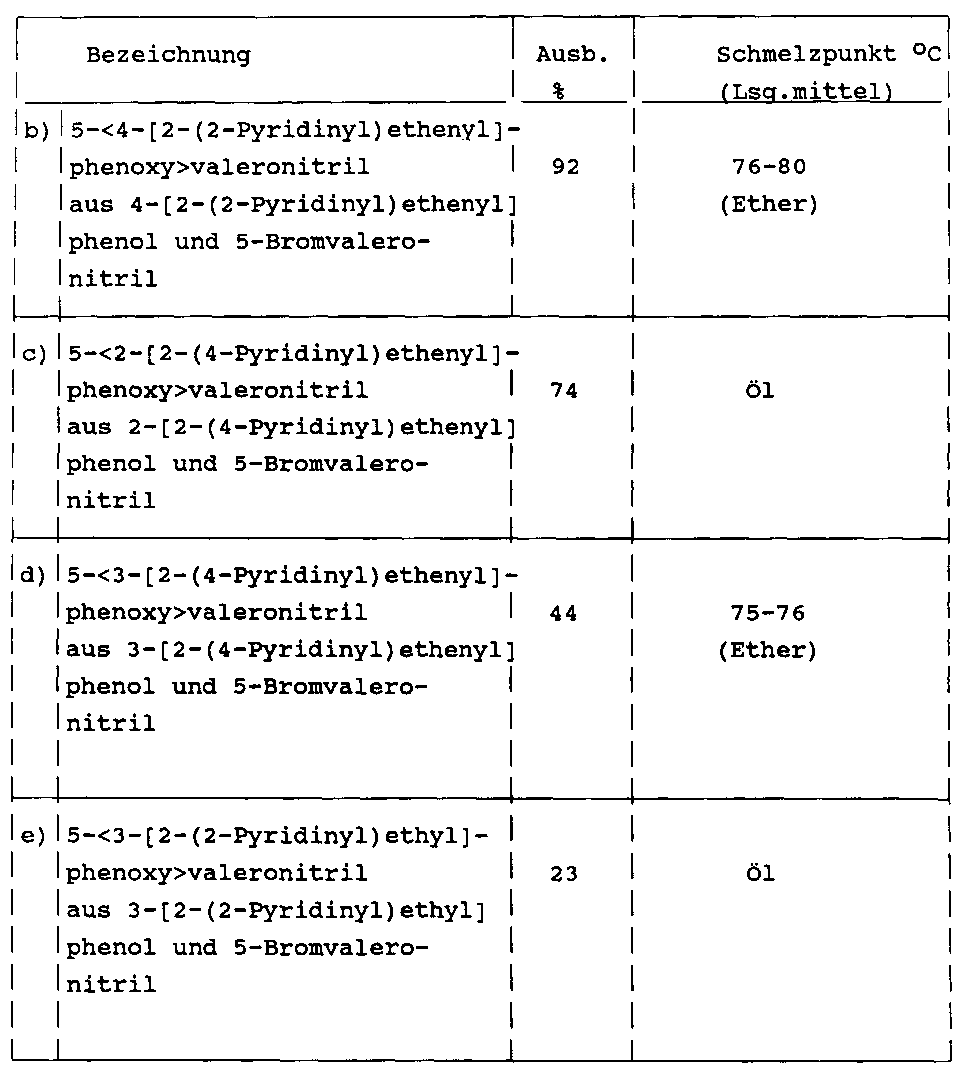

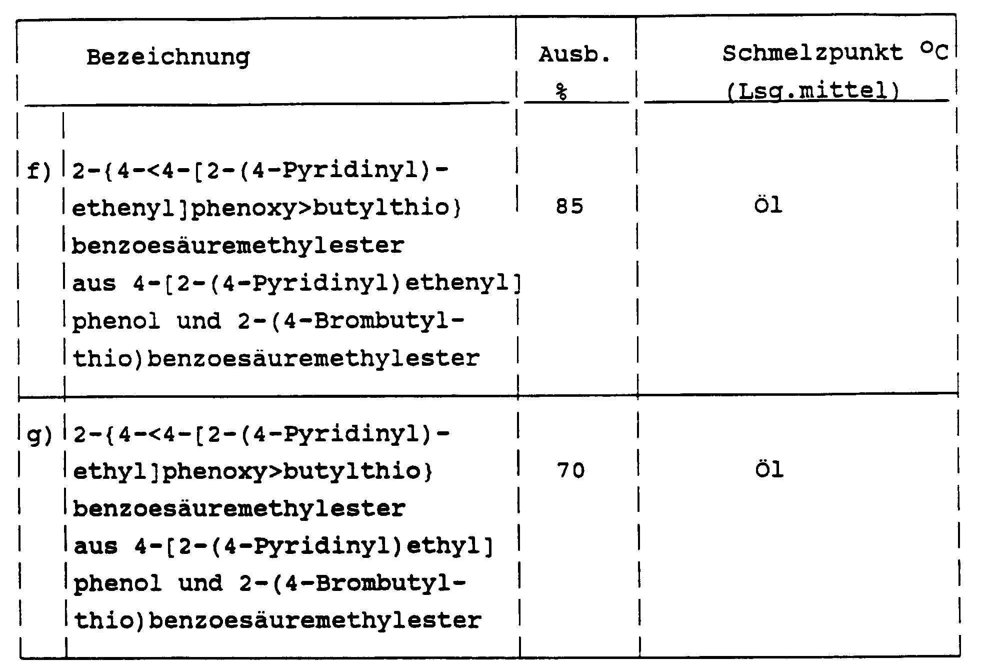

### Beispiel 3

### 4-[2-(2-Pyridinyl)ethenyl]benzonitril

Eine Mischung aus 20.4 g (0.2 mol) Essigsäureanhydrid, 18.6 g (0.2 mol) 2-Methylpyridin und 26.2 g (0.2 mol) 4-Cyanbenzaldehyd wird 24 h zum Rückfluß erhitzt. Man läßt abkühlen, versetzt mit überschüssigem Ether und filtriert. Man isoliert 18.8 g Titelverbindung (46 % d. Th.) vom Schmp. 96-98°C.

### Beispiel 4

### 4-<4-[2-(4-Pyridinyl)ethenyl]phenoxy>buttersäureethylester

Eine Mischung aus 30 g (0.15 mol) 4-[2-(4-Pyridinyl)ethenyl]phenol, 21 g Kaliumcarbonat, 750 ml Butanon und 33 ml (0.23 mol) 4-Brombuttersäureethylester erhitzt man 24 h zum Rückfluß, filtriert, engt das Filtrat ein, versetzt mit Wasser, extrahiert mit Dichlormethan, trocknet den Extrakt, engt ein und verreibt den Rückstand mit Ether. Es verbleiben 42.3 g Titelverbindung (89 % d. Th.) vom Schmp. 83-85°C.

### Beispiel 5

In analoger Weise wie in Beispiel 4 beschrieben erhält man:

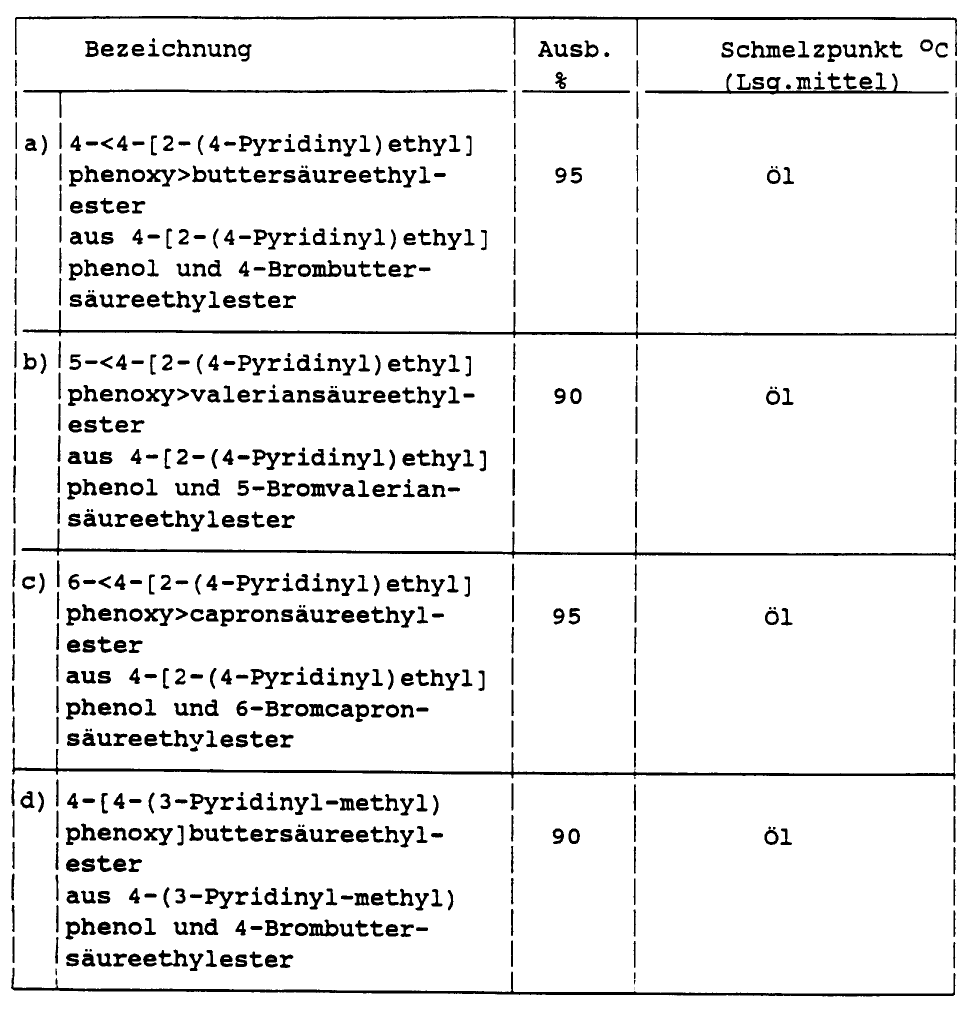

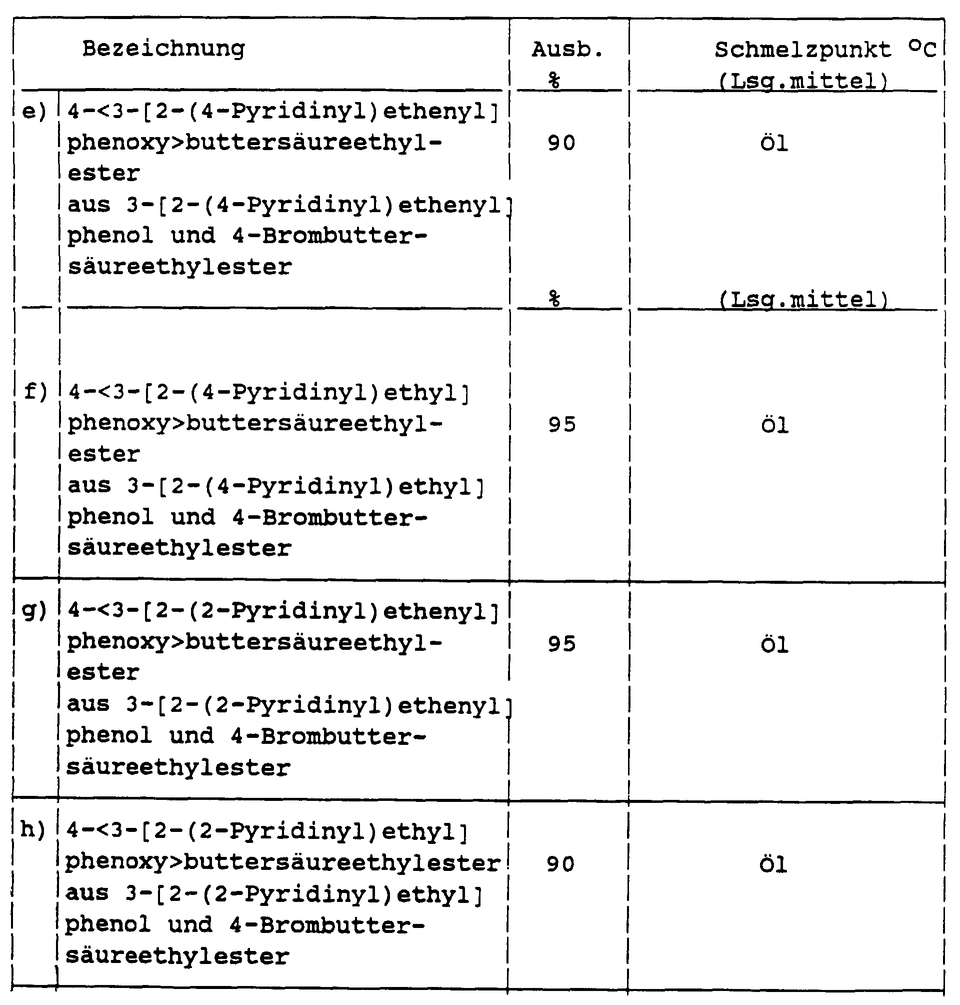

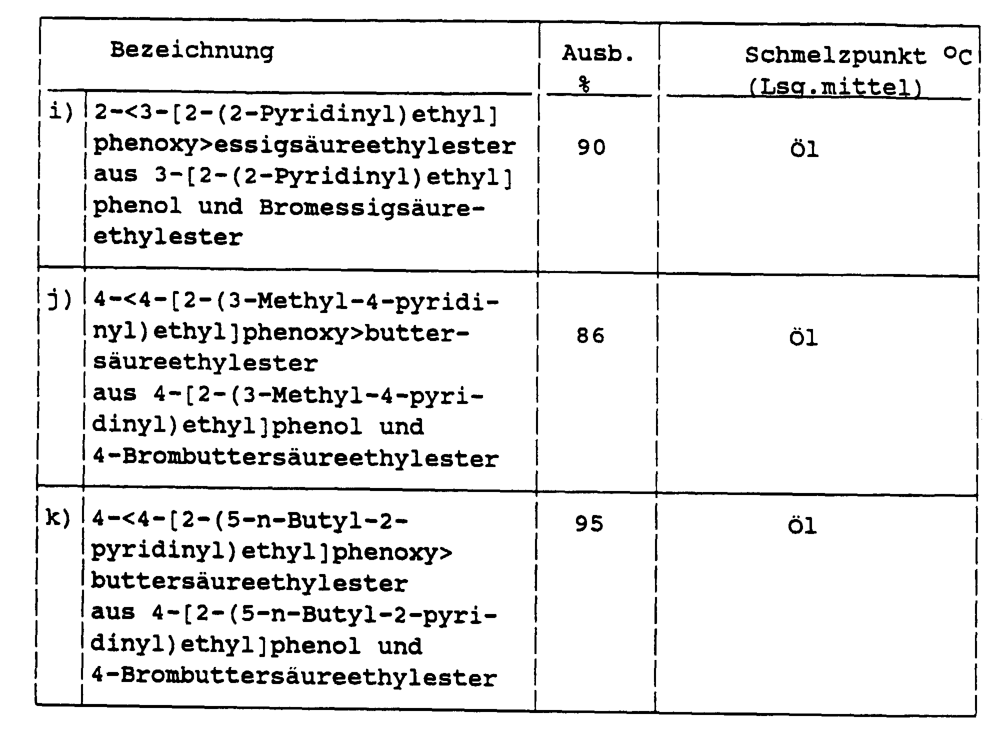

### Beispiel 6

### 5-<2-[2-(2-Pyridinyl)ethenyl]phenoxy>valeriansäureethylester

Zu der Lösung von 2.3 g (0.1 mol) Natrium in 250 ml Ethanol gibt man 19.7 g 2-[2-(2-Pyridinyl)ethenyl]phenol, rührt 10 min bei Raumtemp., fügt 17.4 ml (0.11 mol) 5-Bromvaleriansäureethylester zu und erhitzt 16 h zum Rückfluß. Man engt ein, versetzt mit Wasser, extrahiert mit Dichlormethan, trocknet den Extrakt und engt ein. Es verbleiben 32.4 g Titelverbindung (100 % d. Th.) als Öl.

### Beispiel 7

In analoger Weise wie in Beispiel 6 beschrieben erhält man:

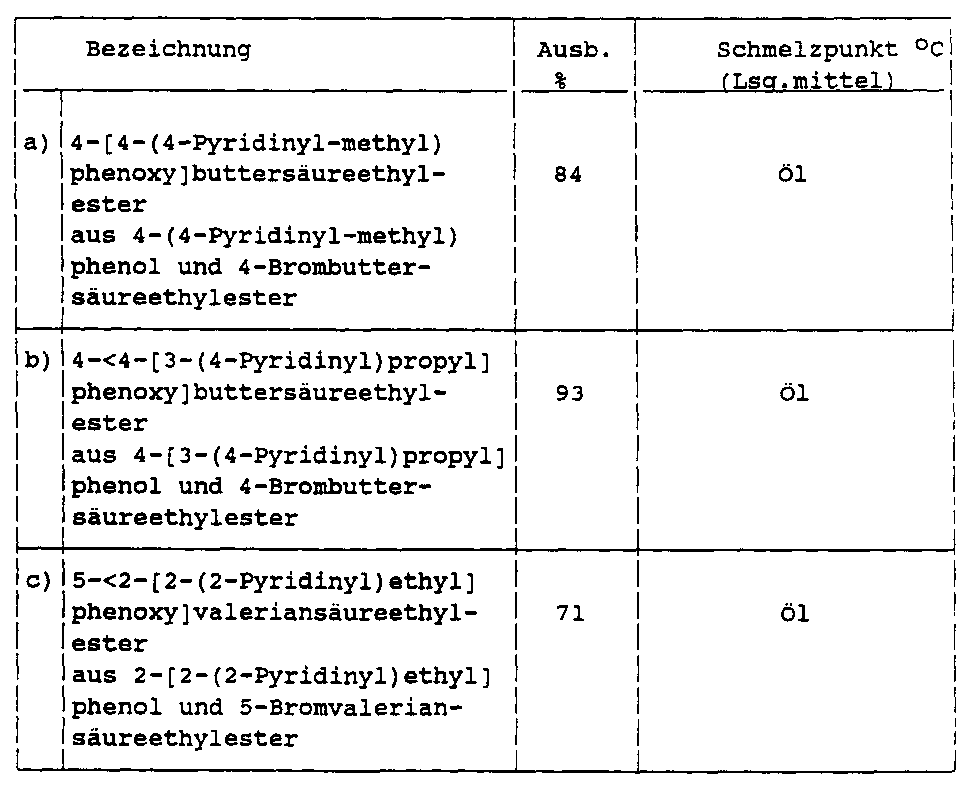

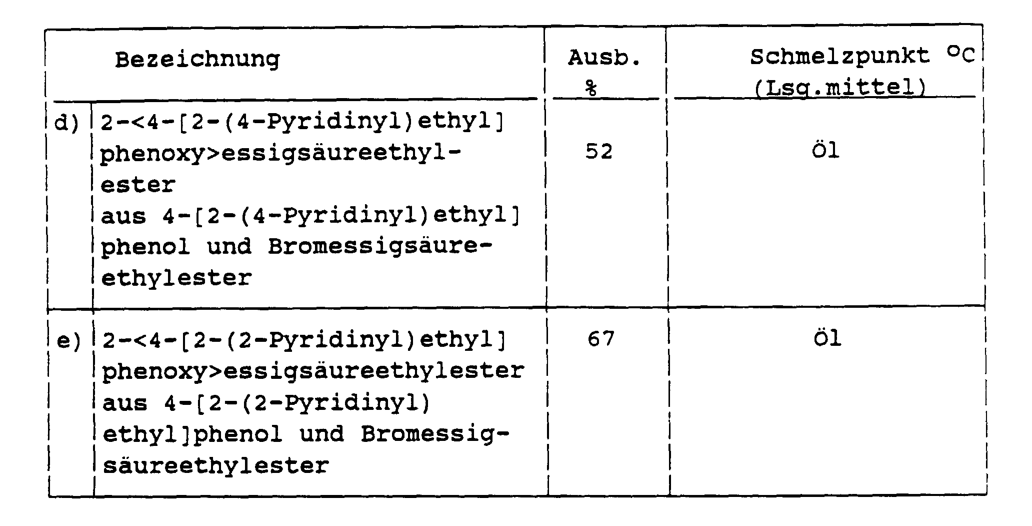

### Beispiel 8

### 6-<4-[2-(4-Pyridinyl)ethyl]phenoxy>capronsäure

Eine Mischung aus 11.3 g (33 mol) Verbindung des Beispiels 5 c und 110 ml 1 N Natronlauge wird 2 h zum Rückfluß erhitzt. Man läßt abkühlen, wäscht mit Ether, stellt die wässrige Phase auf pH 5 ein und filtriert den Niederschlag ab. Man isoliert 8.7 g Titelverbindung (85 % d.Th.) vom Schmp. 180-182°C.

### Beispiel 9

In analoger Weise wie in Beispiel 8 beschrieben erhält man:

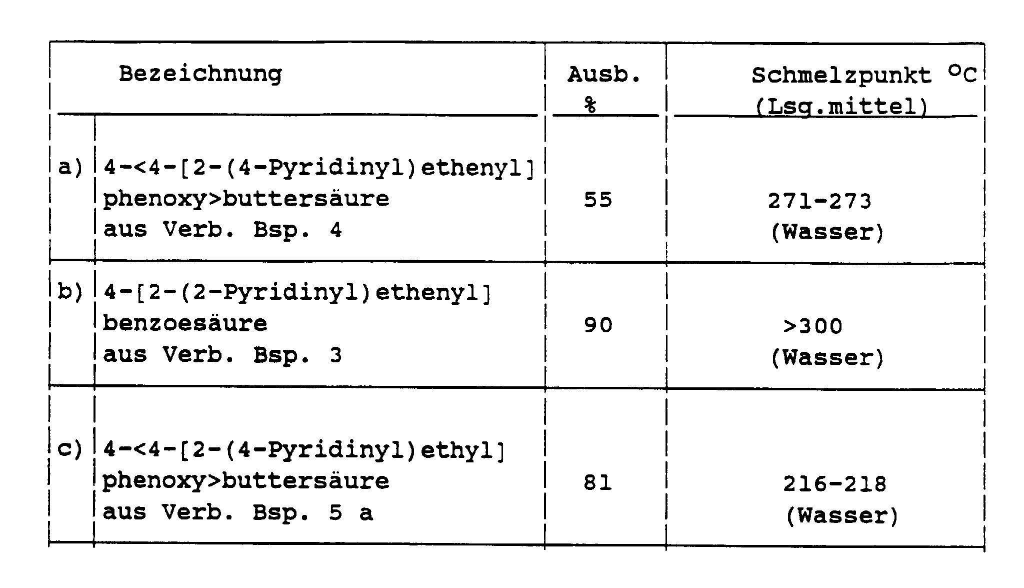

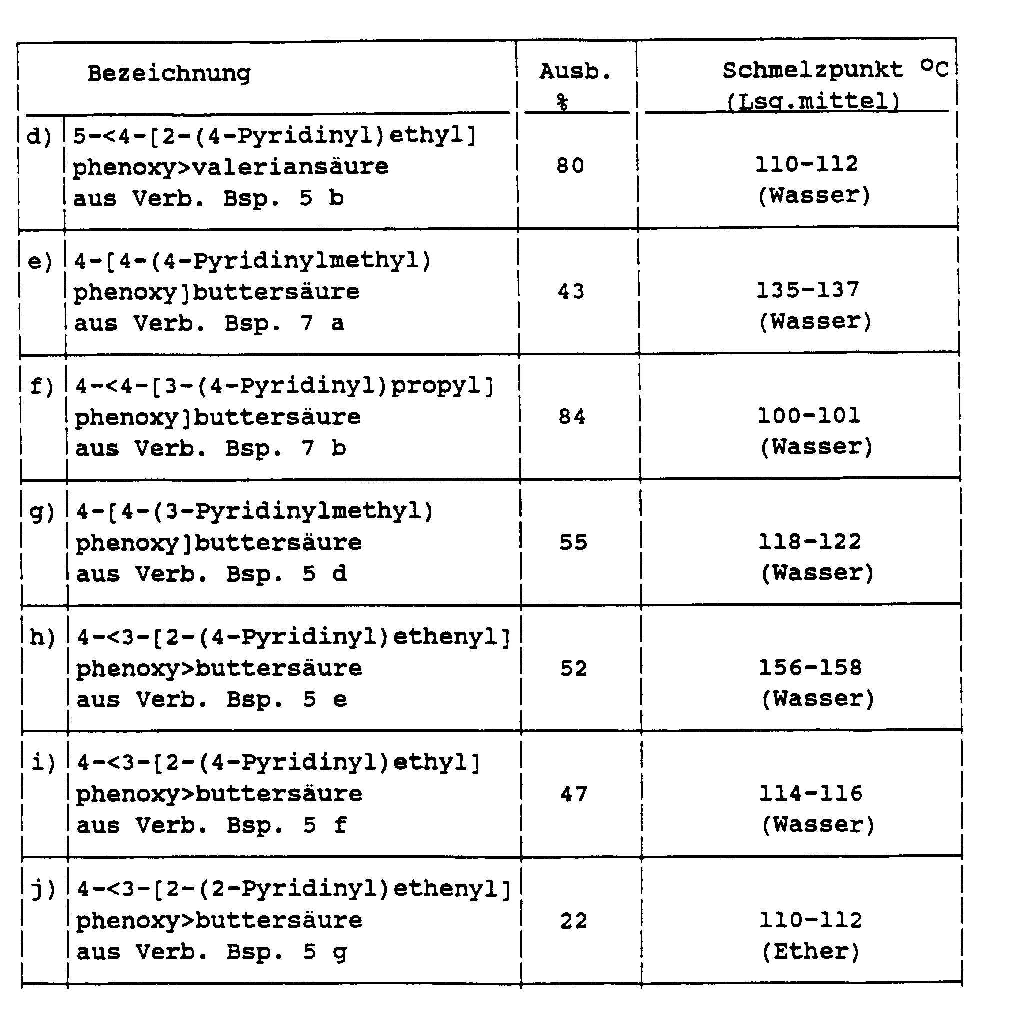

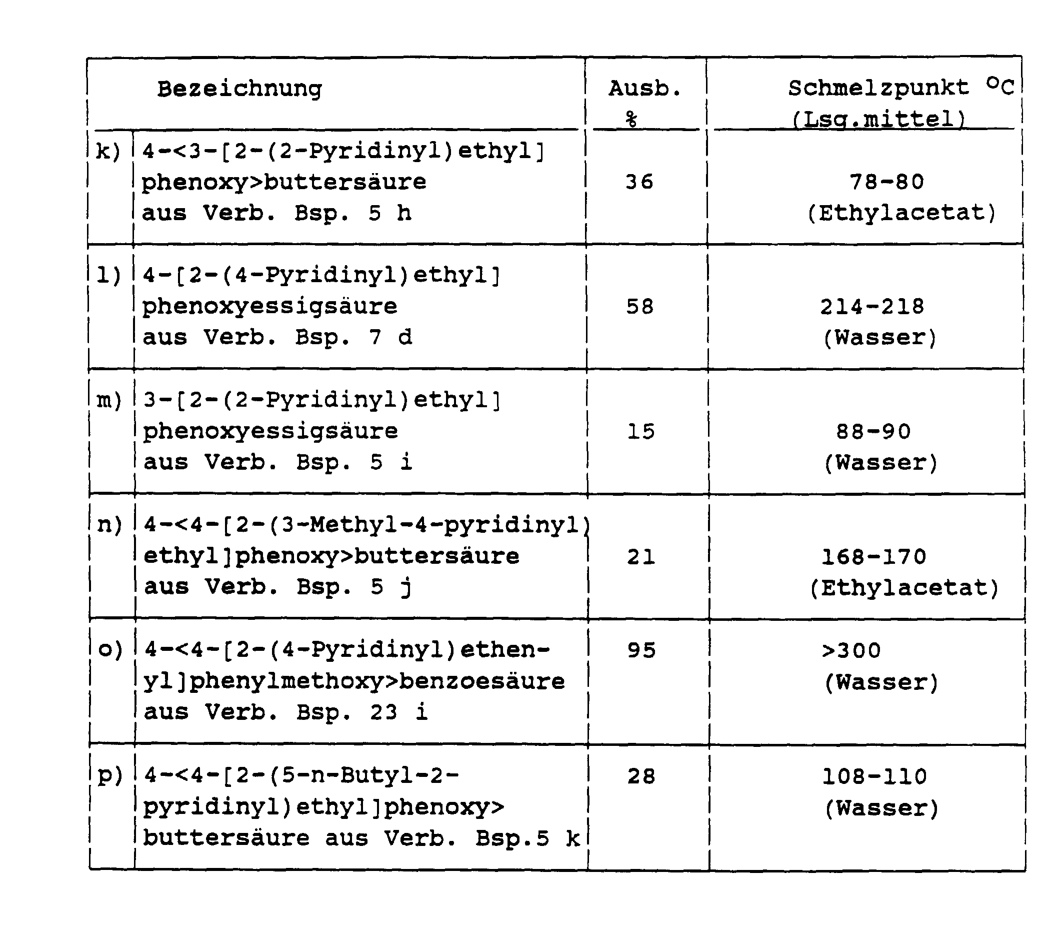

### Beispiel 10

### 5-<2-[2-(2-Pyridinyl)ethenyl]phenoxy>valeriansäure

Eine Mischung aus 32.5 g (0.1 mol) Verbindung des Beispiels 6, 450 ml 50proz. Ethanol und 27 g Kaliumcarbonat wird 2 h zum Rückfluß erhitzt, eingeengt, der Rückstand in Wasser aufgenommen, mit Ether gewaschen, die wässrige Phase auf pH 6.5 eingestellt und der Niederschlag abfiltriert. Man isoliert nach Verreiben mit Ether 12.1 g Titelverbindung (41 % d. Th.) vom Schmp. 90-93°C.

### Beispiel 11

In analoger Weise wie in Beispiel 10 beschrieben erhält man:

| Bezeichnung | | Ausb. % | Schmelzpunkt °C (Lsg.mittel) |
|---|---|---|---|
| a) | 5-<2-[2-(2-Pyridinyl)ethyl]phenoxy>valeriansäure aus Verb. Bsp. 7 c | 75 | 66-68 (Wasser) |
| b) | 2-<4-[2-(2-Pyridinyl)ethyl]phenoxy>essigsäure aus Verb. Bsp. 7 e | 91 | 135-137 (Wasser) |

### Beispiel 12

### 2-{4-<4-[2-(4-Pyridinyl)ethenyl]phenoxy>butylthio}benzoesäure

Eine Mischung aus 5.0 g (12 mmol) Verbindung des Beispiels 2 f, 100 ml Ethanol und 100 ml 1 N Natronlauge wird 1 h zum Rückfluß erhitzt, anschließend abgekühlt, mit Ether gewaschen, die wässrige Phase auf pH 6 eingestellt und der Niederschlag abgesaugt. Nach Verreiben mit Ether verbleiben 2.3 g Titelverbindung (47 % d. Th.) vom Schmp. 229-231°C.

### Beispiel 13

In analoger Weise wie in Beispiel 12 beschrieben erhält man:
2-{4-<4-[2-(4-Pyridinyl)ethyl]phenoxy>butylthio}benzoesäure-hydrochlorid aus Verb. Bsp. 2 g; Ausbeute 85 %, Schmp. 102-104°C (aus Wasser).

### Beispiel 14

### 2-{3-<4-[2-(4-Pyridinyl)ethyl]phenoxy>propylthio}benzosäure

Zu der Lösung von 1.3 g (58 mmol) Natrium in 100 ml Ethanol gibt man 5.7 g (29 mmol) 4-[2-(4-Pyridinyl)ethyl]phenol, rührt 10 min bei Raumtemperatur, fügt 6.7 g (29 mmol) 2-(3-Chlorpropylthio)benzoesäure zu und erwärmt 12 h zum Rückfluß. Man läßt abkühlen, filtriert, engt das Filtrat ein, nimmt in Wasser auf, wäscht mit Ether, stellt die wässrige Phase auf pH 5 ein und extrahiert mit Dichlormethan. Nach Chromatographie des Extrakts an Kieselgel und Verreiben mit Ligroin isoliert man 2.1 g Titelverbindung (19 % d. Th.) vom Schmp. 151-153°C.

### Beispiel 15

### 5-{4-<4-[2-(4-Pyridinyl)ethenyl]phenoxy>butyl}-1H-tetrazol

Eine Mischung aus 5.3 g (19 mmol) Verbindung des Beispiels 1, 3.7 g (57 mmol) Natriumazid, 3.0 g (57 mmol) Ammoniumchlorid und 40 ml N,N-Dimethylformamid wird 3 Tage bei 125°C gerührt. Man fügt noch 2.5 g Natriumazid und 2.0 g Ammoniumchlorid zu, rührt weitere 6 h bei 125°C, engt ein, nimmt den Rückstand in Wasser auf, extrahiert mit Dichlormethan, trocknet, engt ein und verreibt mit 2-Propanol. Es verbleiben 2.7 g Titelverbindung (44 % d. Th.) vom Schmp. 158-159°C.

### Beispiel 16

In analoger Weise wie in Beispiel 15 beschrieben erhält man:

| Bezeichnung | | Ausb. % | Schmelzpunkt °C (Lsg.mittel) |
|---|---|---|---|
| a) | 5-{4-<4-[2-(4-Pyridinyl)ethenyl]phenoxymethyl>phenyl}-1H-tetrazol aus Verb. Bsp. 2 a | 44 | 205-207 (Wasser) |
| b) | 5-{4-<4-[2-(2-Pyridinyl)ethenyl]phenoxy>butyl}-1H-tetrazol aus Verb. Bsp. 2 b | 50 | 152-154 (Wasser) |
| c) | 5-{4-<2-[2-(4-Pyridinyl)ethenyl]phenoxy>butyl}-1H-tetrazol aus Verb. Bsp. 2 c | 35 | 161-163 (Ether) |
| d) | 5-{4-<3-[2-(4-Pyridinyl)ethenyl]phenoxy>butyl}-1H-tetrazol aus Verb. Bsp. 2 d | 35 | 132-135 (Ether) |
| e) | 5-{4-<3-[2-(2-Pyridinyl)ethyl]phenoxy>butyl}-1H-tetrazol aus Verb. Bsp. 2 e | 28 | Öl |
| f) | 5-(4-<4-[2-(4-Pyridinyl)ethenyl]phenylmethoxy>phenyl)-1H-tetrazolnatrium aus Verb. Bsp. 21 | 61 | 264-266 (Ethanol) |

### Beispiel 17

### 4-<4-[2-(4-Pyridinyl)ethenyl]phenoxy>buttersäure-N-(1H-tetrazol-5-yl)amid

Zu einer Lösung von 5.6 g (20 mmol) Verbindung des Beispiels 9a in 40 ml N,N-Dimethylformamid gibt man 3.6 g (22 mmol) N,N'-Carbonyl-diimidazol, rührt 1 h bei 100°C, fügt 1.8 g (22 mmol) 5-Amino-1H-tetrazol zu, rührt 3 h bei 100°C, gießt in Wasser, filtriert ab, suspendiert den Niederschlag in heißem Wasser und stellt auf pH 6 ein. Man filtriert und erhält 3.5 g Titelverbindung (50 % d. Th.) vom Schmp. 274-276°C.

### Beispiel 18

In analoger Weise wie in Beispiel 17 beschrieben erhält man:

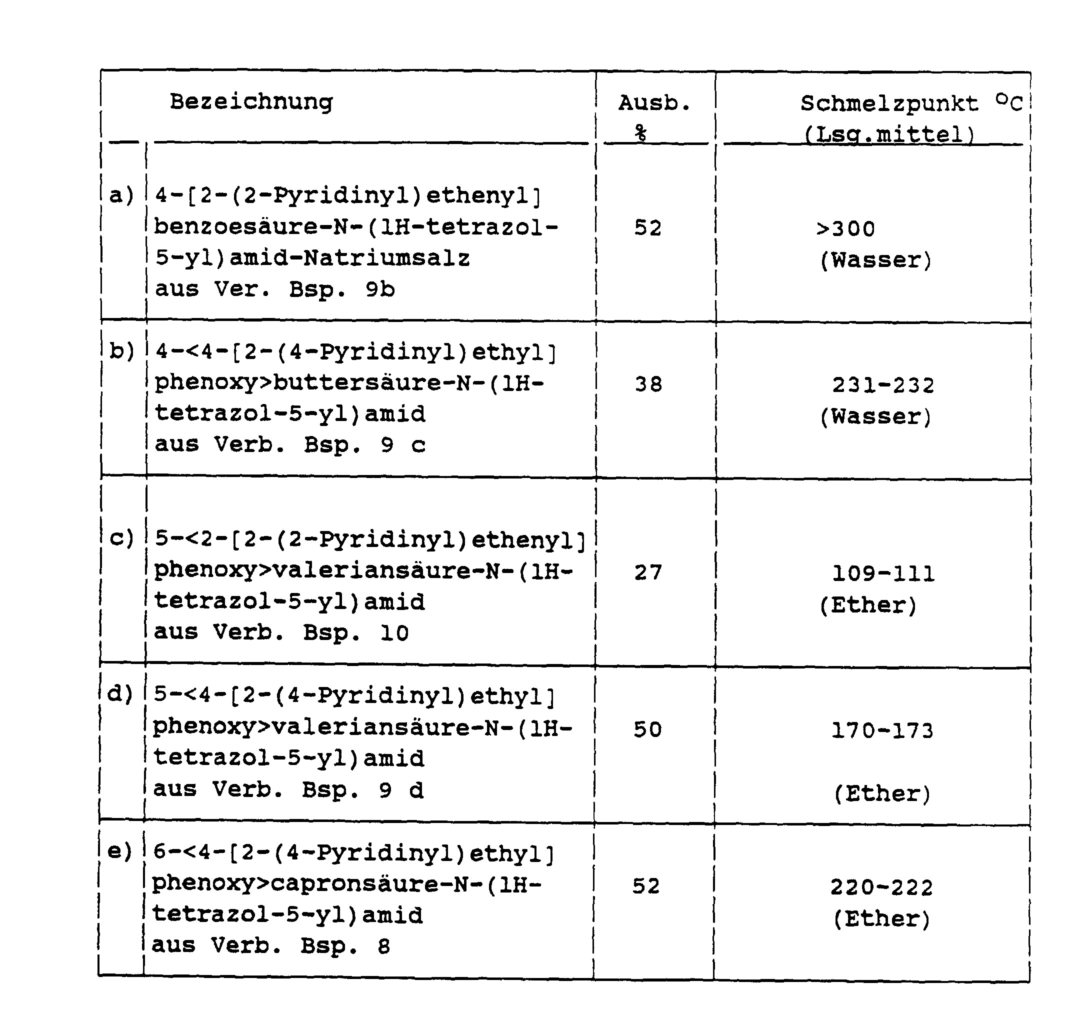

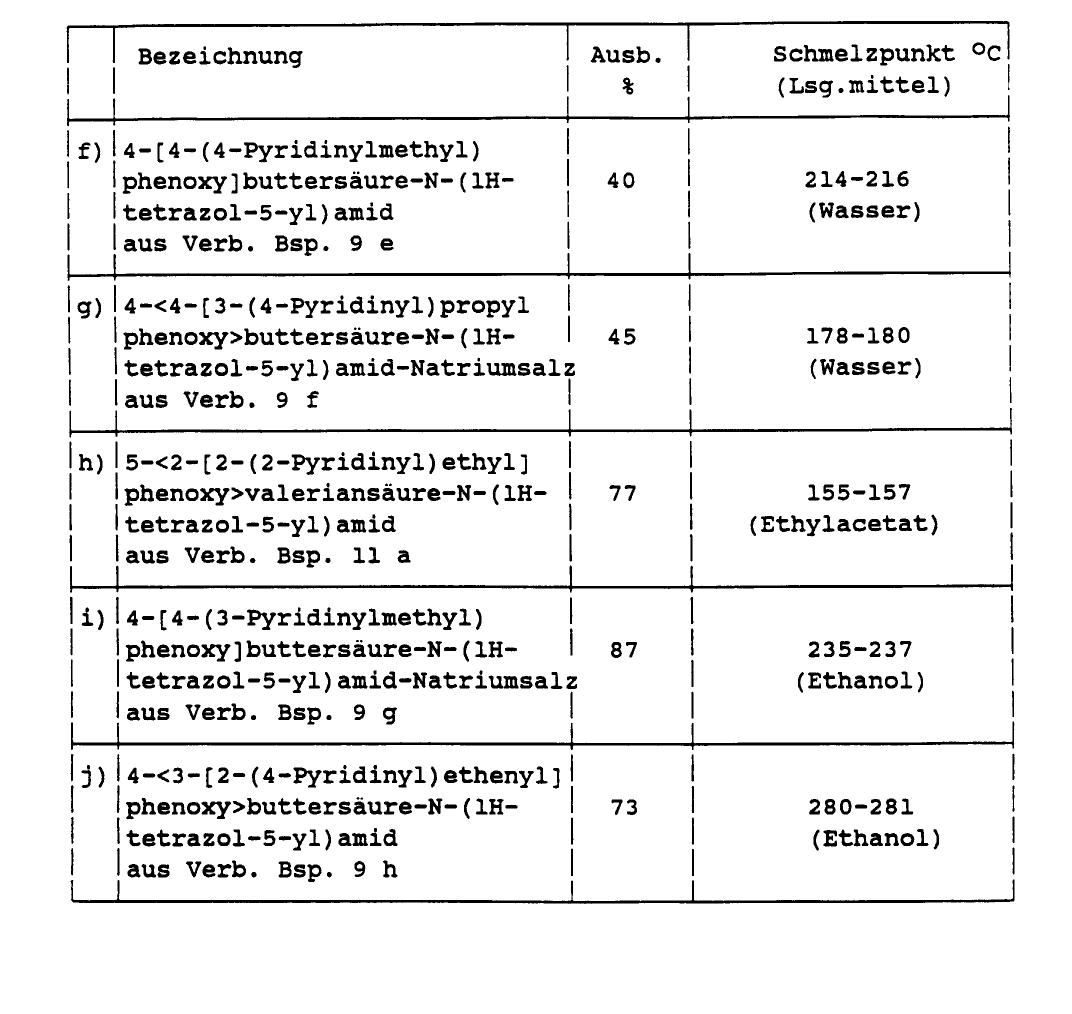

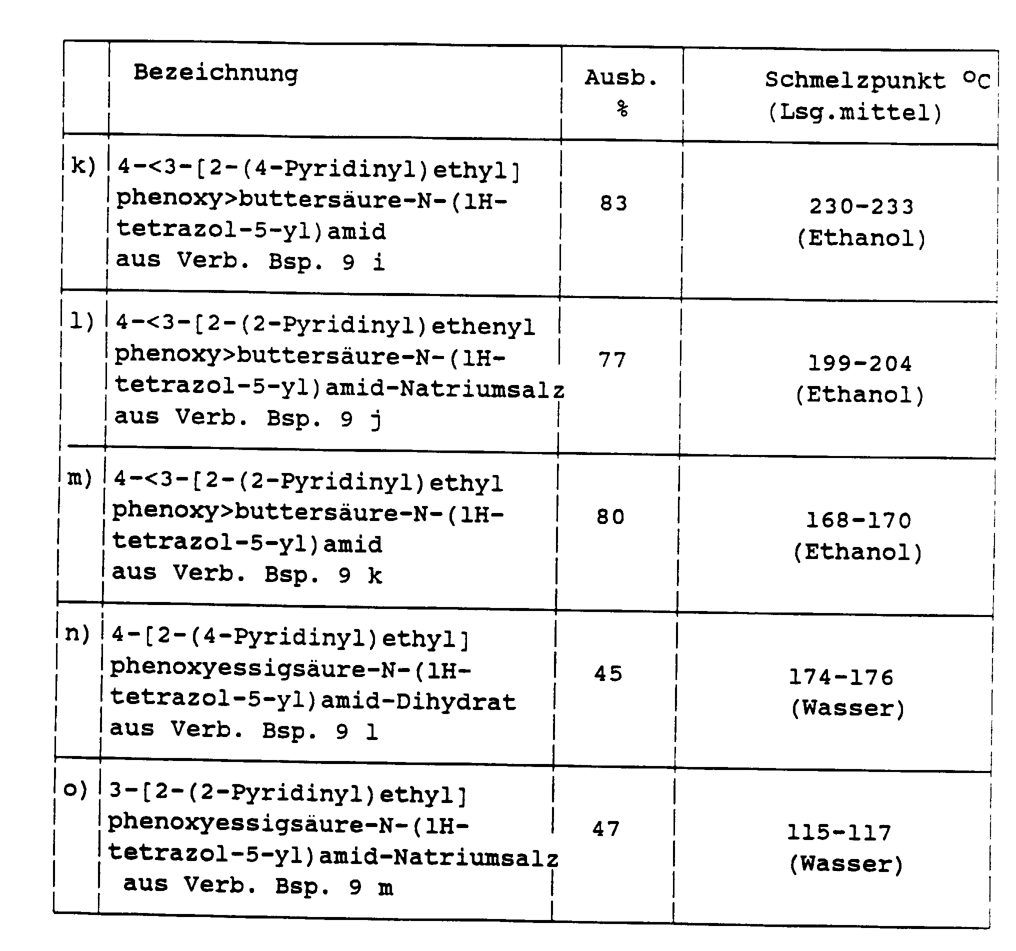

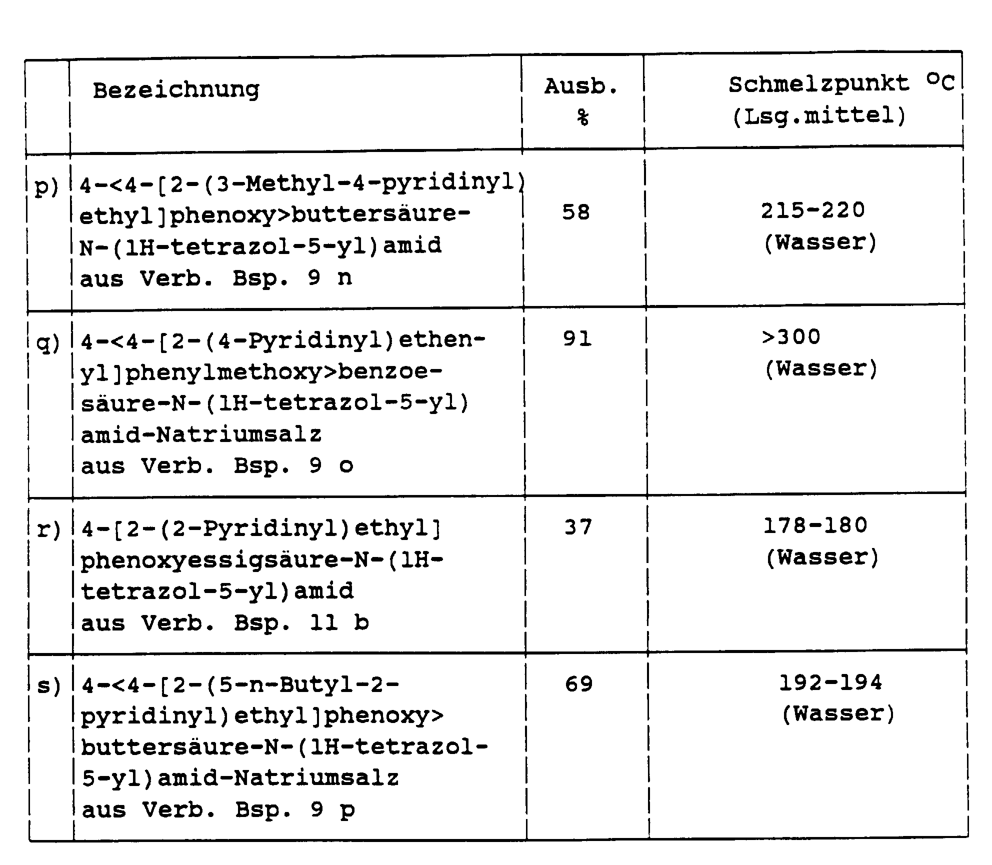

### Beispiel 19

### N-Hydroxy-N-methyl-4-[2-(2-pyridinyl)ethenyl]benzamid

Zu einer Lösung von 4.5 g (20 mmol) Verbindung des Beispiels 9 b in 150 ml Dichlormethan und 2.8 ml Triethylamin tropft man bei -10°C eine Lösung aus 2.6 ml (20 mmol) Chlorameisensäureisobutylester und 20 ml Dichlormethan, rührt 30 min nach, tropft eine Lösung aus 1.7 g (36 mmol) N-Methyl-hydroxylamin, 2.8 ml Triethylamin und 60 ml Dichlormethan zu, rührt 30 min nach, filtriert, wäscht das Filtrat mit Natriumhydrogencarbonatlösung, extrahiert mit verd. Natronlauge und stellt den Extrakt auf pH 8 ein. Nach Extraktion mit Dichlormethan, Trocknen, Einengen und Verreiben mit Ether erhält man 1.7 g Titelverbindung (33 % d.Th.) vom Schmp. 135-138°C.

### Beispiel 20

In analoger Weise wie in Beispiel 19 beschrieben erhält man:

| Bezeichnung | | Ausb. % | Schmelzpunkt °C (Lsg.mittel) |
|---|---|---|---|
| a) | N-Hydroxy-N-methyl-4-<4-[2-(4-pyridinyl)ethenyl]phenoxy>buttersäureamid aus Verb. Bsp. 9 a | 24 | 140-142 (Ether) |
| b) | N-Hydroxy-N-methyl-4-<4-[2-(4-pyridinyl)ethyl]phenoxy>buttersäureamid aus Verb. Bsp. 9 c | 28 | 90-93 (Ether) |

### Beispiel 21

### 4-<4-[2-(4-Pyridinyl)ethenyl]phenylmethoxy>benzonitril

Eine Lösung aus 9.8 g (40 mmol) 4-(4-Formyl-phenylmethoxy)benzonitril, 4 ml (40 mmol) 4-Methyl-pyridin und 4 ml Essigsäureanhydrid wird 18 h auf 120°C erhitzt. Man versetzt mit Wasser, extrahiert mit Dichlormethan, trocknet den Extrakt, engt ein und verreibt mit Ether. Man isoliert 6.1 g Titelverbindung (49 % d.Th.) vom Schmp. 149-150°C.

### Beispiel 22

### 4-<4-[2-(4-Pyridinyl)ethenyl]phenylmethoxy>benzonitril

Eine Mischung aus 2.3 g (10 mmol) 4-[2-(4-Chlormethylphenyl)ethenyl]pyridin, 1.2 g (10 mmol) 4-Hydroxy-benzonitril, 1.4 g Kaliumcarbonat und 10 ml Butanon wird 18 h zum Rückfluß erhitzt, filtriert, das Filtrat eingeengt, in Ethylacetat aufgenommen, mit Wasser gewaschen, getrocknet und eingeengt. Man erhält 2.0 g Titelverbindung (64 % d.Th.) vom Schmp. 147-149°C.

### Beispiel 23

In analoger Weise wie in Beispiel 21 beschrieben erhält man:

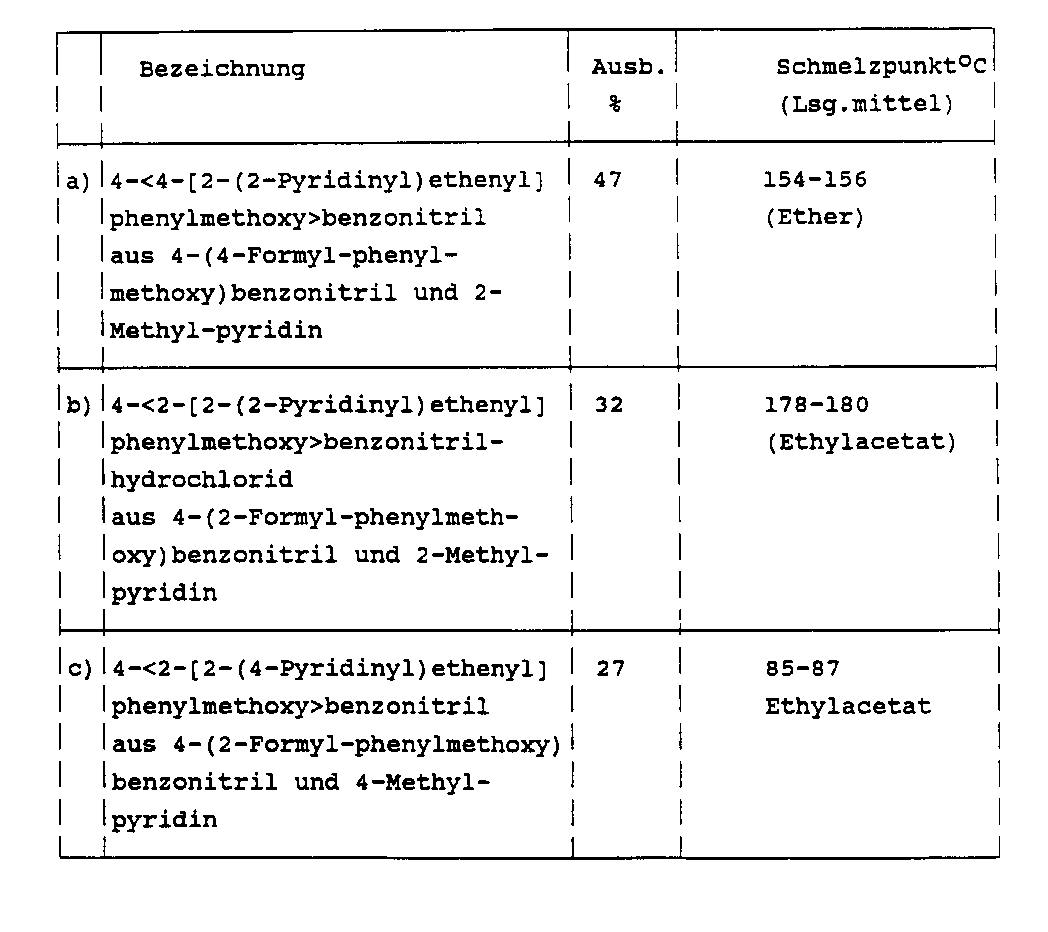

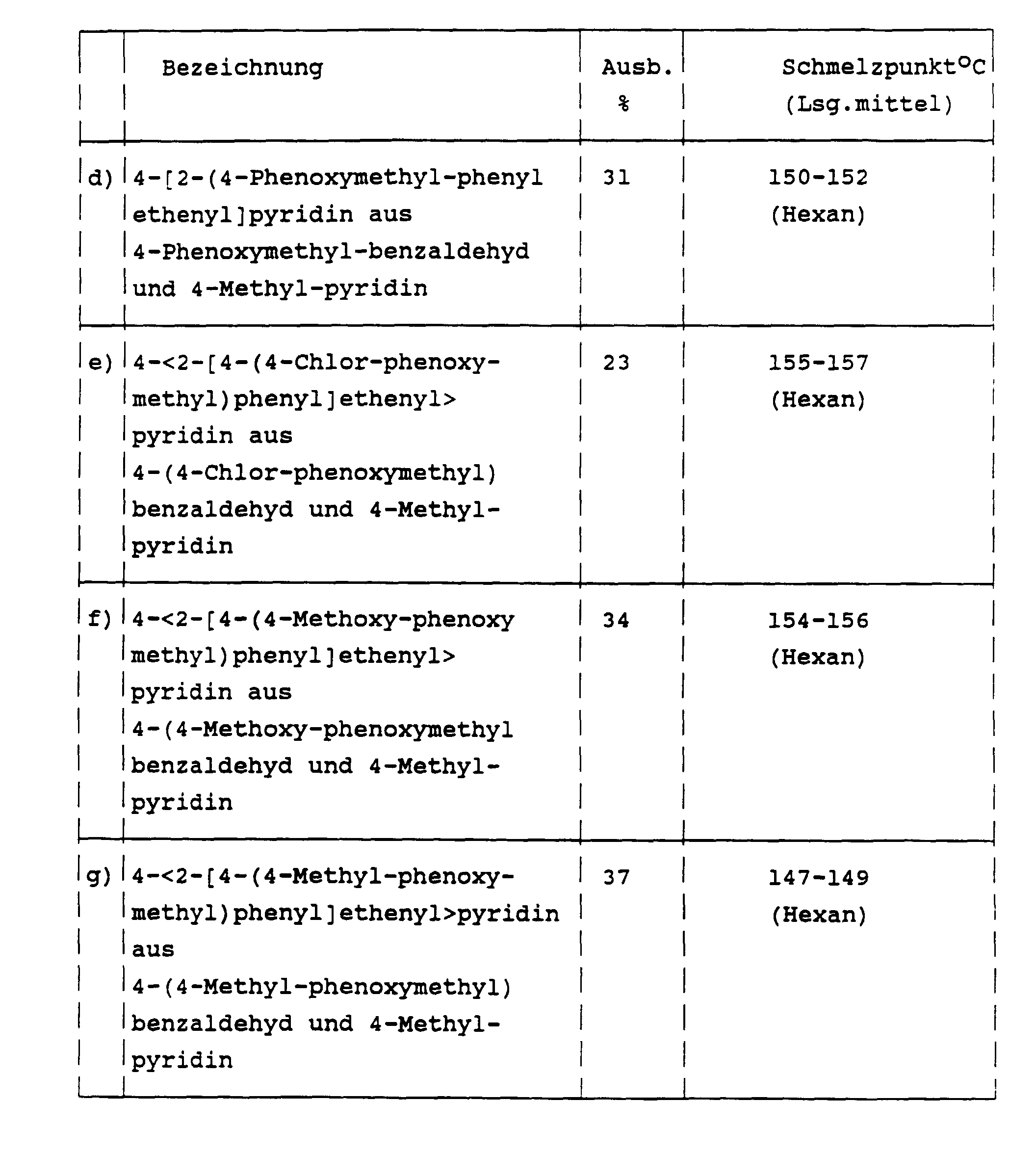

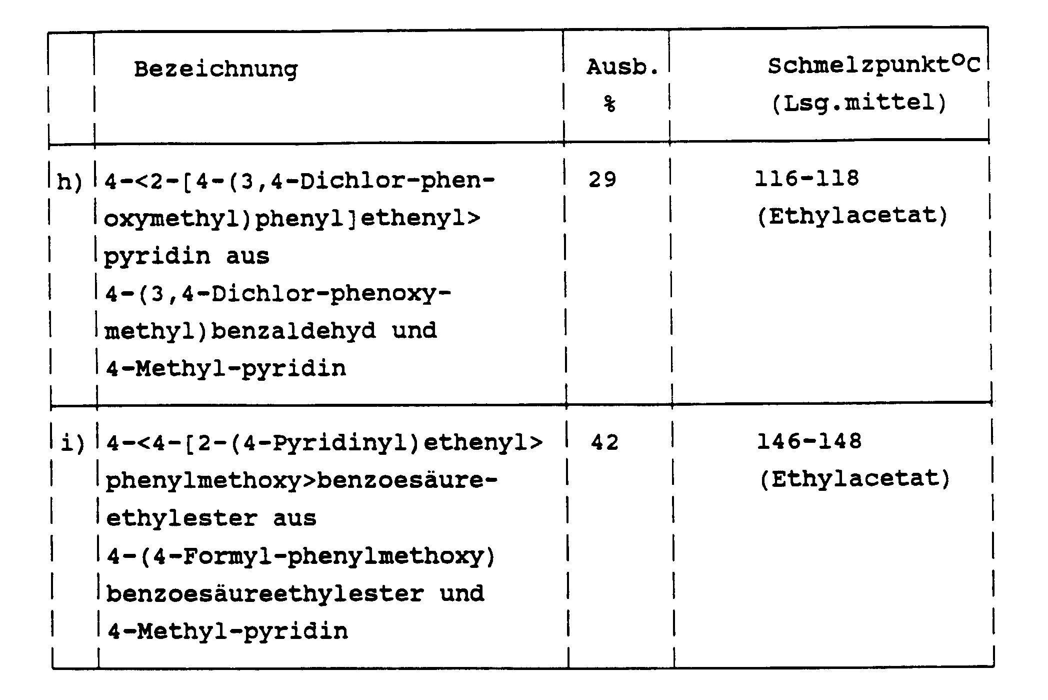

### Beispiel 24

### 4-<4-[2-(4-Pyridinyl)ethyl]phenylmethoxy>benzonitril

Eine Mischung aus 2.0 g (6.4 mmol) Verb. Bsp. 21, 30 ml Ethanol, 60 ml Tetrahydrofuran und 0.2 g 10proz. Palladiumkohle wird bei Raumtemp. und 1 bar Wasserstoffdruck hydriert. Nach Aufnahme der theoretischen Menge wird filtriert, eingeengt und an Kieselgel (Elutionsmittel Isohexan/Ethylacetat) chromatographiert.

Man erhält 1.3 g Titelverbindung (65 % d. Th.) vom Schmp. 139-140° C.

### Beispiel 25

### Hemmung der Antigen-bedingten Konstriktion von passiv sensibilisierten Meerschweinchen-Lungenparenchym-Streifen in vitro (Organbad)

Für die in vitro Untersuchung der erfindungsgemäßen Verbindungen wurde die Hemmung der Antigen-bedingten Konstriktion von passiv sensibilisiertem Meerschweinchen-Lungenparenchym-Streifen gemessen, wie nachfolgend beschrieben:

Pribright-White Meerschweinchen wurden betäubt durch Genickschlag und entblutet. Die Lungen wurden in situ mit Krebs-Puffer, pH 7,4, weitgehend blutfrei gespült.

Anschließend wurde die Lunge entnommen, in Streifen geschnitten (ca. 20 x 4 x 4 mm), und die Streifen für eine Stunde bei Raumtemperatur mit einer 1:50-Verdünnung eines homologen anti-Ov-Albumin-Antiserums passiv sensibilisiert und dann mit Krebs-Puffer lx gewaschen.

Das Antiserum war vorher nach DAVIES in Meerschweinchen des gleichen Stammes erzeugt worden durch wiederholte Injektion von Ov-Albumin (2 x kristallisiert) unter Zusatz von komplettem Freund'schem Adjuvans.

Bis zu seiner Verwendung wurde das Antiserum bei -18° C unverdünnt gelagert.

Anschließend wurden die Lungenstreifen einzeln in 10 ml Wasserbädern mit einer Vorspannung von 1.2 g an einem isometrischen Meßaufnehmer aufgehängt.

Danach wurden die Bäder mit Krebs-Puffer gefüllt und kontinuierlich bei 37° C mit O₂ (95%) und CO₂ (5%) begast. Über einen Verstärker wurden die Konstriktionen der Lungenstreifen auf einem Schreiber aufgezeichnet.

Nach 30-minütiger Eingewöhnungsphase wurden Histamin-Kontrollspasmen zur Erkennung der Reaktionsfähigkeit der Organstücke erzeugt, gewaschen, anschließend für 20 Minuten die Prüfsubstanz bei 37° C vorinkubiert und danach die Ov-Albuminbedingte Konstriktion ausgelöst,.

Die Hemmwirkungen der erfindungsgemäßen Verbindungen wurden als prozentuale Verringerung der Konstriktionsamplitude der "Proben mit Prüfsubstanz" im Verhältnis zu den "unbehandelten Kontrollkonstriktionen" ausgedrückt.

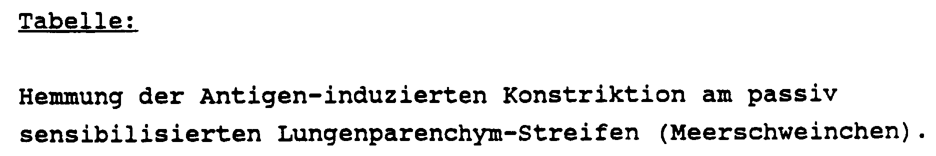

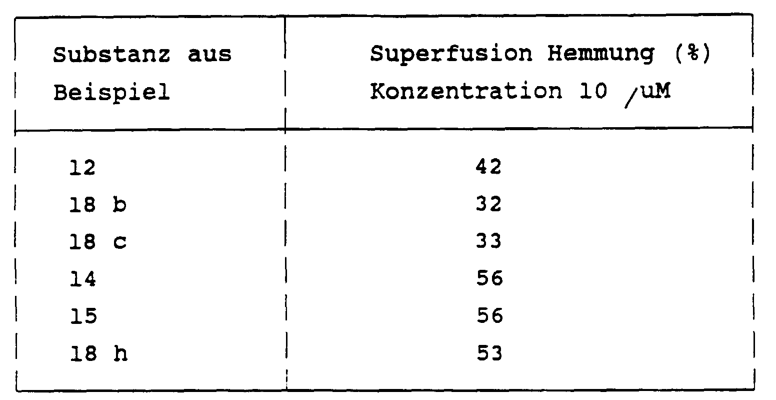

## Patentansprüche

1. Verbindungen der allgemeinen Formel I 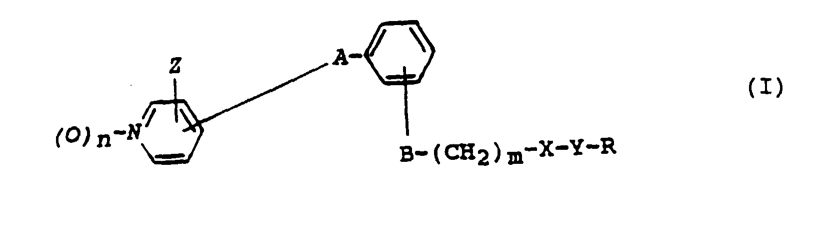 in welcher
A eine Gruppe -CH=CH-,
B ein Sauerstoffatom oder eine Valenzbindung,
m eine ganze Zahl von 0 bis 5,
n die Zahl 0 oder 1,
X eine Valenzbindung, ein Sauerstoffatom oder ein Schwefelatom,
Y eine Valenzbindung,
Z Wasserstoff, Halogen, C₁- bis C₆-Alkyl oder Cyano und
R eine Gruppe CN, COOH, COOC₁- bis C₆-Alkyl, CONH-Tetrazolyl, CON(OH)(C1-C6-Alkyl) oder 5-(1H)-Tetrazolyl bedeuten,
mit Ausnahme der Verbindung 4-[2-(4-Pyridinovinyl)-carboxymethoxybenzol, mit der Maßgabe, daß wenn R eine Gruppe CN, CO-NH-Tetrazolyl, CO-N(OH)(C₁-C₆-Alkyl) oder 5-(1H)-Tetrazolyl und B, m, n, X, Y und Z die angegebene Bedeutung haben, A auch einen Alkylenrest mit 1 bis 3 Kohlenstoffatomen darstellen kann,
sowie deren physiologisch verträgliche Salze.

2. Verbindungen der Formel I 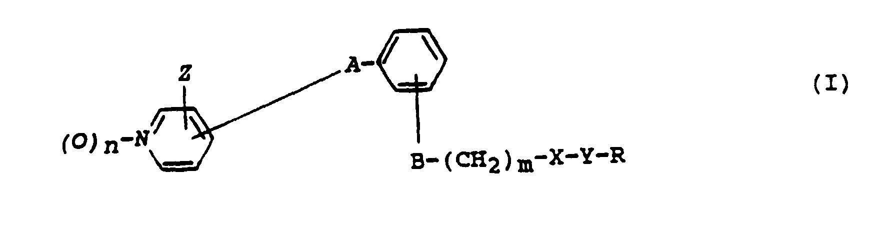 in welcher
A einen Alkylenrest mit 1 bis 3 Kohlenstoffatomen oder eine Gruppe -CH=CH-,
B ein Sauerstoffatom oder eine Valenzbindung,
m eine ganze Zahl von 0 bis 5,
n die Zahl 0 oder 1,
X eine Valenzbindung, ein Sauerstoffatom oder ein Schwefelatom,
Y einen gewünschtenfalls ein- oder mehrfach durch C₁-C₆-Alkyl, C₁-C₆-Alkoxy, Halogen, Hydroxy oder Aminocarbonyl substituierten Phenylenrest,
Z Wasserstoff, Halogen, C₁- bis C₆-Alkyl oder Cyano und
R eine Gruppe CN, COOH, COOC₁- bis C₆-Alkyl, CONH-Tetrazolyl, CON(OH)(C₁-C₆-Alkyl) oder 5-(1H)-Tetrazolyl bedeuten,
sowie deren physiologisch verträgliche Salze.

3. Verfahren zur Herstellung von Verbindungen gemäß Anspruch 1
dadurch gekennzeichnet, daß man in an sich bekannter Weise entweder
a) eire Verbindung der allgemeinen Formel II 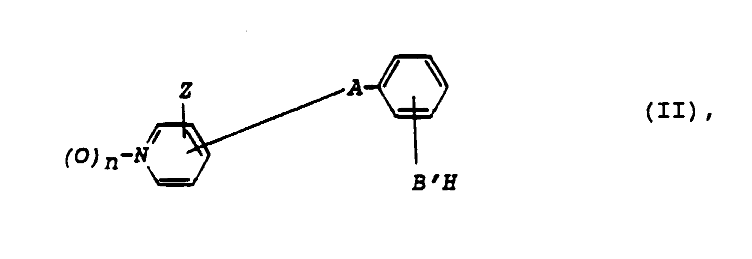 in welcher
A, Z und n die angegebene Bedeutung haben und B' ein Sauerstoffatom bedeutet, mit einer Verbindung der allgemeinen Formel III
G-(CH₂)ₘ-X-Y-R (III),
in welcher
X, Y, R und m die angegebene Bedeutung haben und G einen reaktiven Rest darstellt,
umsetzt, oder
b) eine Verbindung der allgemeinen Formel IV 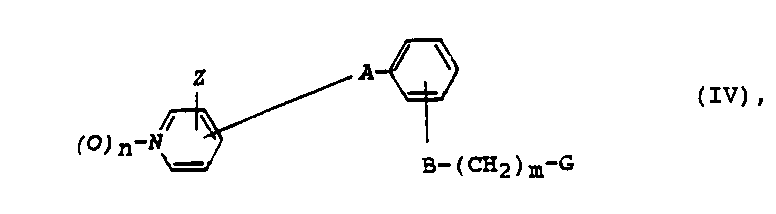 in welcher A, B, G, Z, m und n die angegebene Bedeutung haben,
mit einer Verbindung der allgemeinen Formel V
H-X-Y-R (V),
in welcher
R, X und Y die angegebene Bedeutung haben,
umsetzt, oder
c) für den Fall, daß A die Gruppe -CH=CH- bedeutet, eine Verbindung der allgemeinen Formel VI 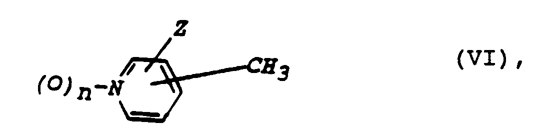 in welcher
Z und n die angegebene Bedeutung haben,
mit einer Verbindung der allgemeinen Formel VII 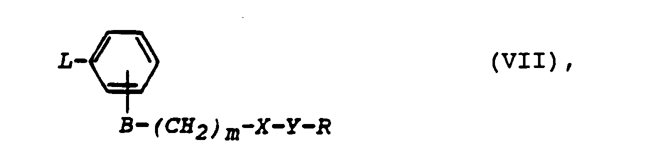 in welcher
B, X, Y, R und m die angegebene Bedeutung haben und L eine Formylgruppe oder ein reaktives Derivat hiervon darstellt,
umsetzt,
und anschließend gewünschtenfalls einen Substituenten R in einen anderen, durch die Definition gegebenen Substituenten R oder eine Gruppe A in eine andere durch die Definition gegebene Gruppe A überführt oder den Pyridinstickstoff oxidiert und die erhaltene Verbindung der Formel I gewünschtenfalls durch Umsetzung mit physiologisch verträglichen Säuren oder Basen in ein Salz umwandelt.

4. Verfahren gemäß Anspruch 3 zur Herstellung von Verbindungen gemäß Anspruch 2

5. Arzneimittel, enthaltend mindestens eine Verbindung gemäß Anspruch 1 oder 2, neben üblichen Träger- und Hilfsstoffen.

6. Verwendung von Verbindungen gemäß Anspruch 1 oder 2 zur Herstellung von Arzneimitteln zur Behandlung von allergischen Krankheiten.

## Claims

1. Compounds of the general formula I 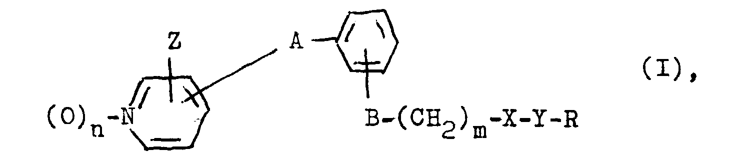 in which A signifies a group -CH=CH-, B an oxygen atom or a valency bond, m a whole number from 0 to 5, n the number 0 or 1, X a valency bond, an oxygen atom or a sulphur atom, Y a valency bond, Z hydrogen, halogen, C₁- to C₆-alkyl or cyano and R a group CN, COOH, COOC₁ to C₆-alkyl, CONH-tetrazolyl, CON(OH)(C₁-C₆-alkyl) or 5-(1H)-tetrazolyl, with the exception of the compound 4-[2-(4-pyridinovinyl)-carboxymethoxybenzene, with the proviso that when R is a group CN, CO-NH-tetrazolyl, CO-N(OH)(C₁-C₆-alkyl or 5-(1H)-tetrazolyl and B, m, n, X, Y and Z have the given meaning, A can also represent an alkylene radical with 1 to 3 carbon atoms, as well as their physiologically compatible salts.

2. Compounds of the formula I 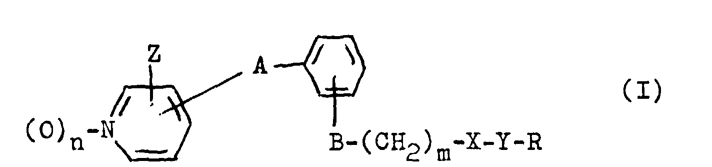 in which A signifies an alkylene radical with 1 to 3 carbon atoms or a group -CH=CH-, B an oxygen atom or a valency bond, m a while number of 0 to 5, n the number 0 or 1, X a valency bond, an oxygen atom or a sulphur atom, Y a phenylene radical substituted one or more times by C₁-C₆-alkyl, C₁-C₆-alkoxy, halogen, hydroxyl or aminocarbonyl, Z hydrogen, halogen, C₁- to C₆-alkyl or cyano and R a group CN, COOH, COOC₁- to C₆-alkyl, CONH-tetrazolyl, CON(OH)(C₁-C₆-alkyl) or 5-(1H)-tetrazolyl, as well as their physiologically compatible salts.

3. Process for the preparation of compounds according to claim 1, characterised in that, in per se known manner, one either
a) reacts a compound of the general formula II 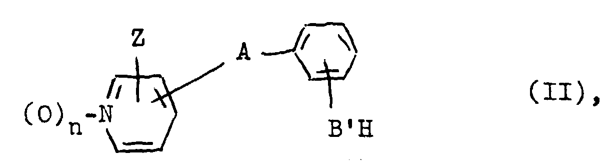 in which A, Z and n have the given meaning and B' signifies an oxygen atom, with a compound of the general formula III
G-(CH₂)ₘ-X-Y-R (III),
in which X, Y, R and m have the given meaning and G represents a reactive residue, or
b) reacts a compound of the general formula IV 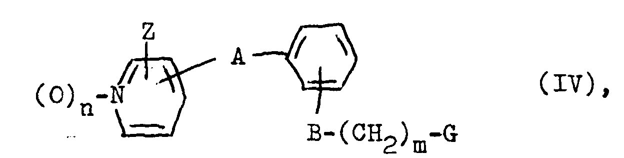 in which A, B, G, Z, m and n have the given meaning, with a compound of the general formula V
H-X-Y-R (V)
in which R, X and Y have the given meaning, or
c) for the case that A signifies the group -CH=CH-, reacts a compound of the general formula VI 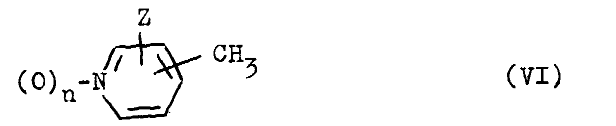 in which Z and n have the given meaning, with a compound of the general formula VII 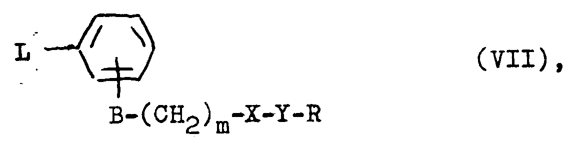 in which B, X, Y, R and m have the given meaning and L represents a formyl group or a reactive derivative hereof,
and subsequently, if desired, converts a substituent R into another substituent R given by the definition or a group A into another group A given by the definition or oxidises the pyridine nitrogen and, if desired, converts the compound obtained of the formula I into a salt by reaction with physiologically compatible acids or bases.

4. Process according to claim 3 for the preparation of compounds according to claim 2.

5. Medicaments containing at least one compound according to claim 1 or 2, besides usual carrier and adjuvant materials.

6. Use of compounds according to claim 1 or 2 for the preparation of medicaments for the treatment of allergic diseases.

## Revendications

1. Composés de formule générale I 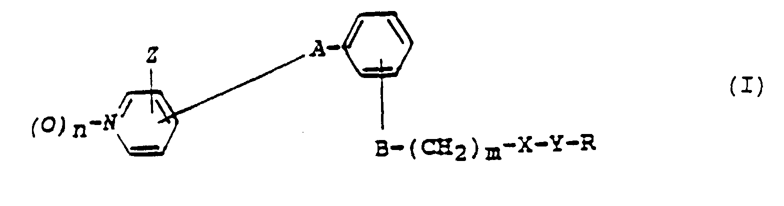 dans laquelle
A représente un groupe -CH=CH-,
B représente un atome d'oxygène ou une valence libre,
m représente un nombre entier de 0 à 5,
n vaut 0 ou 1,
X représente une valence libre, un atome d'oxygène ou un atome de soufre,
Y représente une valence libre,
Z représente un atome d'hydrogène, d'halogène, un groupe alkyle en C₁ à C₆ ou cyano et
R représente un groupe CN, COOH, COO(alkyle en C₁ à C₆), CONH-tétrazolyle, CON(OH)(alkyle en C₁ à C₆) ou 5-(1H)-tétrazolyle,
à l'exception du composé 4-[2-(4-pyridinovinyl)-carboxyméthoxybenzène, étant entendu que dans le cas où R représente un groupe CN, CO-NH-tétrazolyle, CO-N(OH)(alkyle en C₁ à C₆) ou 5-(1H)-tétrazolyle et B, m, n, X, Y et Z ont les significations indiquées, A peut représenter également un reste alkylène ayant 1 à 3 atomes de carbone,
ainsi que leurs sels physiologiquement acceptables.

2. Composés de formule générale I 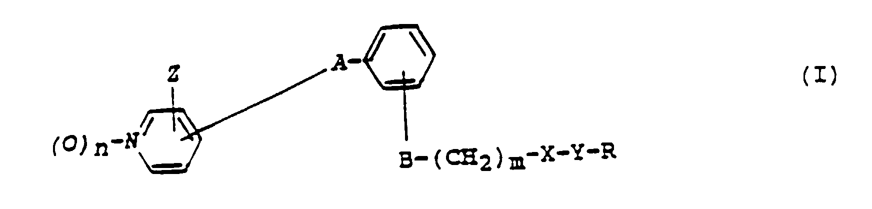 dans laquelle
A représente un reste alkylène ayant 1 à 3 atomes de carbone ou un groupe -CH=CH-,
B représente un atome d'oxygène ou une valence libre,
m représente un nombre entier de 0 à 5,
n vaut 0 ou 1,
X représente une valence libre, un atome d'oxygène ou un atome de soufre,
Y représente un reste phénylène éventuellement substitué une ou plusieurs fois par un groupe alkyle en C₁ à C₆, alcoxy en C₁ à C₆, halogéno, hydroxy ou aminocarbonyle,
Z représente un atome d'hydrogène, d'halogène, un groupe alkyle en C₁ à C₆ ou cyano et
R représente un groupe CN, COOH, COO(alkyle en C₁ à C₆), CONH-tétrazolyle, CON(OH)(alkyle en C₁ à C₆) ou 5-(1H)-tétrazolyle,
ainsi que leurs sels physiologiquement acceptables.

3. Procédé de préparation de composés selon la revendication 1, caractérisé en ce que, de manière connue en soi, soit
a) on fait réagir un composé de formule générale II 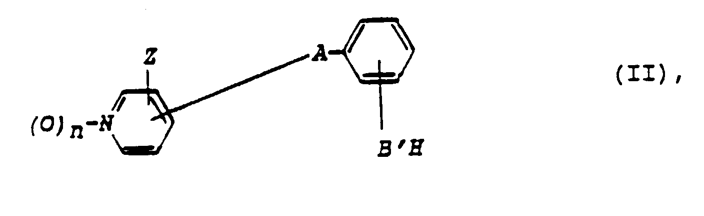 dans laquelle
A, Z et n ont les significations indiquées et B' représente un atome d'oxygène, avec un composé de formule générale III
G-(CH₂)ₘ-X-Y-R (III),
dans laquelle X, Y, R et m ont les significations mentionnées et G représente un reste réactif, soit
b) on fait réagir un composé de formule générale IV 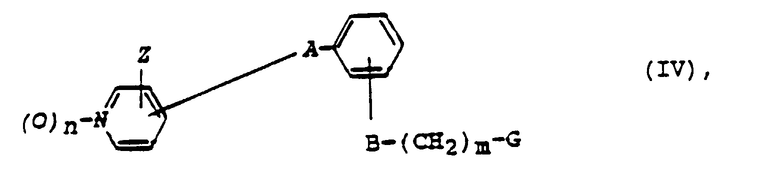 dans laquelle A, B, G, Z, m et n ont les significations indiquées,
avec un composé de formule générale V
H-X-Y-R (V),
dans laquelle
R, X et Y ont les significations mentionnées, soit
c) dans le cas où A représente le groupe -CH=CH-, on fait réagir un composé de formule générale VI 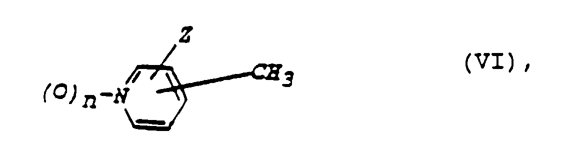 dans laquelle
Z et n ont les significations mentionnées,
avec un composé de formule générale VII 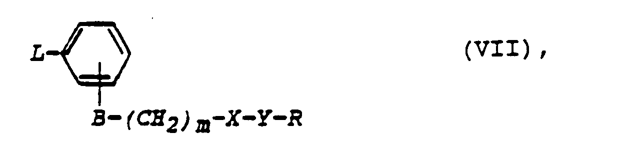 dans laquelle
B, X, Y, R et m ont les significations mentionnées et L représente un groupe formyle ou un dérivé réactif de celui-ci,
et ensuite, le cas échéant, on transforme un substituant R en un autre substituant R répondant à la définition ou on transforme un groupe A en un autre groupe A répondant à la définition ou on oxyde l'azote pyridinique et on transforme éventuellement le composé de formule I obtenu en un sel par réaction avec des acides ou bases physiologiquement acceptables.

4. Procédé selon la revendication 3 pour la préparation de composés selon la revendication 2.

5. Médicament contenant au moins un composé selon la revendication 1 ou 2, en plus de véhicules et adjuvants usuels.

6. Utilisation de composés selon la revendication 1 ou 2, pour la préparation de médicaments destinés au traitement de maladies allergiques.
